# EUROPEAN PATENT APPLICATION

(11) **EP 0 818 539 A1**
(43) Date of publication of application: **14.01.1998**
(21) Application number: 96111156.4
(22) Date of filing: 11.07.1996
(51) Int. Cl.: C12P 1/06, C12R 1/045, A61K 38/04

(54) **Methylsulfomycin I, a process for its production and its use**

(71) Applicant: HOECHST AKTIENGESELLSCHAFT, 65929 Frankfurt am Main (DE)
(72) Inventor: Nadkarni, Suresh Rudra, Dr., Bombay 400 080 (IN); Mukhopadhyay, Triptikumar, Dr., Bombay 400 082 (IN); Jayvanti, Kenia, Dr., Bombay 400 080 (IN); Vijaya Kumar, Erra Koteswara, Dr., Bombay 400 082 (IN)

(57) **Abstract**

Methylsulfomycin I, a compound of the formula C₅₅H₅₄N₁₆O₁₆S₂, has an antibiotic activity.

## Description

This invention relates to a methylsulfomycin I, a process for its production and its use. Methylsulfomycin I may be described as an antibacterial antibiotic belonging to the class of cyclic thiopeptides. Cyclic thiopeptide antibiotics are narrow-spectrum antibiotics acting mainly against Gram positive bacteria. Several cyclic thiopeptides are reported in literature like sulfomycins I, II and III [J. Antibiotics, 22, 12-17 (1969); Tetrahedron letters, 29, 1401-1404 (1988)]; thioxamycin [J. Antibiotics, 42, 1465-1469 (1989)]; A 10255B, A10255G and A 10255J, [J. Org. Chem, 57, 5200-5208 (1992)] and berninamycin A [J. Am. Chem. Soc., 99, 1645-1646 (1977); Tetrahedron letters, 29, 1401-1404 (1988).

However, methylsulfomycin I described herein differs from all the known cyclic thiopeptide antibiotics in its molecular formula and structure and therefore forms the subject of this invention. Furthermore, a chemical abstract on-line search performed with the search keys of molecular weight and molecular formula confirms the novelty of the compound.

Methylsulfomycin I can be produced by the cultivation of the microorganism HIL 006044 or its mutants or variants.

The microorganism culture number HIL 006044, used for the production of methylsulfomycin I, was isolated from a soil sample collected in Greece. The microorganism, HIL 006044 belongs to the order Actinomycetales. It has been deposited under the conditions of the Treaty of Budapest with Deutsche Sammlung von Mikroorganismen GmbH, Mascheroder Weg 1b, D-38124 Braunschweig on June 18, 1996 (DSM 11008).

The said cultivation of culture HIL 006044, its mutants and variants is carried out, under aerobic conditions in a nutrient medium containing sources of carbon and nitrogen, nutrient inorganic salts and trace elements followed by isolation and purification of the said antibiotic from the culture filtrate and mycelium.

The preferred carbon sources are starch, glucose, sucrose, dextrin, fructose, molasses, glycerol, lactose or galactose. A particularly preferred carbon source is starch. The preferred sources of nitrogen are soybean meal, peanut meal, yeast extract, beef extract, peptone, malt extract, corn steep liquor, gelatin or casamino acids. A particularly preferred nitrogen source is soybean meal. Preferred nutrient inorganic salts are sodium hydrogen phosphate, potassium hydrogen phosphate, sodium chloride, calcium chloride, calcium carbonate, potassium nitrate, ammonium sulphate or magnesium sulphate. Trace elements could be for example salts of iron, manganese, copper, zinc or cobalt or such other heavy metals.

Cultivation of culture No. HIL 006044 is preferably carried out at temperatures between 28-32°C and pH between 6.0 and 8.0. Particularly preferred is the cultivation at 29°C ( 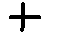 1°C) and pH about 7.0.

The fermentation is preferably carried out for 60 to 72 hours when an optimal yield of methylsulfomycin I is obtained. It is particularly preferred to carry out the fermentation for 68-72 hours under submerged conditions for example in shake flasks as well as in laboratory fermenters. If desired, an antifoam agent, such as, "Desmophen®" may be used in the fermenters. The progress of fermentation and formation of the methylsulfomycin I can be detected by High Pressure Liquid Chromatography (HPLC) and by measuring the bioactivity of the culture broth against Staphylococci and Enterococci species by the known microbial agar plate diffusion assay method. The preferred culture is Staphylococcus aureus 3066 (S. aureus 3066) known to be resistant to methicillin, a β-lactam antibiotic reported in literature and Enterococcus faecium R-2 (E. faecium R-2) which is resistant to vancomycin and teicoplanin.

In the resulting culture broth, methylsulfomycin I is present in the culture filtrate as well as the mycelial cake and can be isolated using standard separation techniques. Thus, it can be recovered by extraction of the culture filtrate with a water immiscible solvent such as ethyl acetate, dichloromethane, chloroform or butanol at pH 5-8 or by hydrophobic interaction chromatography using polymeric resins such as Diaion HP-20® (Mitsubishi Chemical Industries Limited, Japan), Amberlite XAD® (Rohm and Haas Industries U.S.A.) activated charcoal or ion-exchange chromatography at pH 5-8. The preferred method is extraction with solvents like ethyl acetate. Concentration and lyophilization gives the crude antibiotic. Methylsulfomycin I can also be recovered from the mycelium by extraction with water miscible solvents such as methanol, acetone or acetonitrile followed by concentration and hydrophobic interaction chromatography over polymeric resins such as Diaion HP-20 or Amberlite XAD or by extraction with water immiscible solvents such as ethyl acetate, dichloromethane chloroform or n-butanol. The preferred method is methanol extraction, followed by concentration.
The crude methylsulfomycin I can be further purified by using any of the following techniques: normal phase chromatography (using alumina or silica gel as stationary phase and eluents such as ethyl acetate, chloroform, methanol or combinations thereof), reverse phase chromatography (using reverse phase silica gel like dimethyloctadecylsilylsilica gel, also called RP-18 or dimethyloctylsilylsilica gel also called RP-8 as stationary phase and eluents such as water, buffers viz. phosphate, acetate, citrate (pH 2-8) and organic solvents methanol, acetonitrile or acetone, or combinations of these solvents), gel permeation chromatography using resins such as Sephadex LH-20® (Pharmacia Chemical Industries, Sweden), in solvents such as methanol, chloroform or ethyl acetate or their combinations; or by counter-current chromatography using a biphasic eluent system made up of two or more solvents such as water and chloroform. These techniques may be used repeatedly or a combination of the different techniques may be used. The preferred method is chromatography over reverse phase modified silica gel (RP-18).

Pharmaceuticals which contain methylsulfomycin I as active substance are subject of the present invention also. They can be prepared by mixing the said compound with one or more pharmacologically tolerated auxiliaries and/or excipients such as, for example, fillers, emulsifiers, lubricants, masking flavors, colorants or buffer substances, and converted into a suitable pharmaceutical form such as, for example, tablets, coated tablets, capsules, or a suspension or solution suitable for parenteral administration.
Examples of auxiliaries and/or excipients which may be mentioned are tragacanth, lactose, talc, agar-agar, polyglycols, ethanol and water. Suitable and preferred for parenteral administration are suspension or solutions in water. It is also possible to administer the active substances as such, without vehicles or diluents, in a suitable form, for example in capsules. Methylsulfomycin I and/or its physiologically tolerated salts can be administered, for example orally, intramuscularly or intravenously.

Suitable doses of the compound of this invention or its physiologically tolerated acid addition salts are about 0.1 to 20 g/day, preferably 0.5 to 4 g/day, for an adult of body weight about 60 kg.

It is possible to administer single doses or, in general, multiple doses, it being possible for the single dose to contain the active substance in an amount of about 50 to 4,000 mg, preferably of about 500 to 2,000 mg.

The instant invention is further characterized by the following examples and by the content of the patent claims.

### EXAMPLE I - Isolation of the culture HIL 006044 from soil

### (a) Composition of nutrient isolation medium

| | |
|---|---|
| Corn starch | 10.0 g |
| Casein | 1.0 g |
| Peptone | 1.0 g |
| Yeast Extract | 1.0 g |
| K₂HPO₄ | 0.5 g |
| Agar powder | 13.0 g |
| Demineralized water | 1.0 litre |
| pH | 7.5 |

### (b) Soil plating and isolation

10 g of soil collected from Greece was added to 90 ml of sterilized demineralized water in 250 ml Erlenmeyer flask which was then shaken for 2 hours on a rotary shaker 220 rpm). The above soil suspension was then serially diluted in steps of 10 up to 10⁻⁵. From the last dilution, 1 ml of suspension was placed at the center of a sterile glass petri plate (15 cms diameter) in which was then poured approximately 50 ml of the above isolation medium supplemented with 25 mcg/ml of amphotericin B as an antifungal agent and cooled to 45°C and the plate swirled thoroughly. The mixture of soil suspension and medium was allowed to settle and incubated at 28°C (± 1°C) for 7 days. The petri plate was periodically observed and the culture No. HIL 006044 was isolated from amongst the growing microorganisms.

### EXAMPLE II - Maintenance of the culture HIL 006044

### Composition of maintenance medium

Culture No. HIL 006044 was maintained on the following medium :

| | |
|---|---|
| Malt Extract | 10.0g |
| Yeast Extract | 4.0 g |
| Glucose | 4.0 g |
| Agar Powder | 13.0 g |
| Demineralized Water | 1.0 litre |
| pH | 7.0 |

After dissolving the above mentioned ingredients throughly by heating, it was distributed in test tubes and then sterilized at 121°C for 20 minutes. The test tubes were then cooled and allowed to solidify in a slanting position. The agar slants were streaked with the growth of the culture No. HIL 006044 by a wire loop and incubated at 28°C (± 1°C) until a good growth was observed. The well grown cultures were stored in the refrigerator at 8°C.

### EXAMPLE III - Fermentation of culture HIL 006044 in shake flasks

### Composition of seed medium :

| | |
|---|---|
| Glucose | 15.0 g |
| Soybean meal | 15.0 g |
| Corn Steep liquor | 5.0 g |
| CaCO₃ | 2.0 g |
| Demineralized water | 1.0 litre |
| pH | 7.0 |

The above seed medium was distributed in 80 ml amounts in 500 ml Erlenmeyer flasks and autoclaved at 121°C for 20 minutes. The flasks were cooled to room temperature and each flask was then inoculated with a loopful of the above mentioned well grown culture of Example II and shaken on a rotary shaker for 72 hours at 240 rpm at 29°C (± 1°C) to give seed culture.

### Composition of the production medium

| | |
|---|---|
| Soluble starch | 25.0 g |
| Glucose | 10.0 g |
| Yeast extract | 2.0 g |
| Soybean meal | 10.0 g |
| CaCO₃ | 1.0 g |
| NaCl | 5.0 g |
| MgSO₄.7H₂O | 1.0 g |
| Demineralized water | 1.0 litre |
| pH | 7.0 |

The production medium was distributed in 60 ml amounts in 500 ml Erlenmeyer flasks and autoclaved at 121°C for 20 minutes. The flasks were cooled to room temperature and then inoculated with the above mentioned seed culture (1 % v/v). The fermentation was carried out on a rotary shaker at 240 rpm and at a temperature of 29°C (± 1°C) for 68 hours.

The production of the antibiotic was monitored by HPLC as well as by testing its bioactivity against S. aureus 3066 and E. faecium R-2 using the well diffusion method in the usual known manner. After harvesting, the culture broth was centrifuged and methylsulfomycin I was isolated from the culture filtrate and mycelium and purified as described in Example V.

### EXAMPLE IV - Cultivation of the culture No. HIL 006044 in fermenters

### Preparation of seed culture in shake flasks

The seed medium of Example III was distributed in 160 ml amounts in 1 L Erlenmeyer flasks and autoclaved for 20 minutes. The seed culture was grown in these flasks as described in Example III.

### Large Scale Fermentation

### Composition of the production medium : As described in Example III.

90 liters of the production medium in 150 litre MNV fermenter along with 45 ml of Desmophen^{(R)} as an antifoam was sterilized in situ for 45 minutes at 121°C, cooled to 28° ± 1°C and seeded with 3 liters of the seed culture mentioned above.

The fermentation was run with the following parameters :

| | |
|---|---|
| Temperature | 29°C (± 0.5°C) |
| Agitation | 110-120 rpm |
| Aeration | 80-100 1pm |
| Harvest time | 69-72 hrs. |

The production of the antibiotic was monitored by HPLC and by testing the bioactivity against S. aureus 3066 and E. faecium R-2. When fermentation was discontinued, the pH of the culture broth was 7.0-7.5. The culture broth was centrifuged after harvesting and the antibiotic methylsulfomycin I was isolated and purified from the culture filtrate and mycelium as described below.

### Example V - Isolation and purification of methylsulfomycin I

Approximately 100 liters of the culture broth were harvested and separated from the mycelium (7.7 kg) by centrifugation. Methylsulfomycin I was found to be present in the culture filtrate as well as the mycelium. The culture filtrate (92 liters) was extracted with ethyl acetate (2 x 45 liters) and the combined ethyl acetate extracts were concentrated under reduced pressure of 10-100 mm of Hg at 35-40°C to obtain crude methylsulfomycin I (25.7 g). The mycelium (7.7 kg) was extracted with methanol (3 x 30 liters). The combined methanol extracts were concentrated under reduced pressure of 10-100 mm of Hg at 35-40°C and lyophilized to get more crude methylsulfomycin I (30.5 g). This was combined with the crude material obtained from culture filtrate and subjected to high pressure liquid chromatography on RP-18 (reverse phase silica gel 50-70µ) packed in a [(32 mm x 250 mm) + (62 mm x 300 mm)] stainless steel column. The column was developed in water and then eluted with water-methanol mixtures where the methanol concentration was increased in steps of 10% up to 50% and then in steps of 2% up to 60% methanol. Thus elution was done with 10% (3 liters) 20% (3 liters), 30% (3 liters), 40% (3 liters), 50% (3 liters), 52% (3 liters), 54% (3 liters), 56% (5 liters), 58% (4 liters) and 60% (3 liters). A flow rate of 100 ml per minute was maintained at a pressure of 150-175 bars. The eluates were collected in fractions of 1 litre and the presence of methylsulfomycin I was monitored by HPLC and also by checking each fraction for their bioactivity against S. aureus 3066 and E. faecium R-2. Methylsulfomycin I eluted out in 50-58% methanol in water system which was then concentrated under reduced pressure of 10 to 100 mm of Hg at 30-40°C and lyophilized to obtain semi-pure methylsulfomycin I (1.1 g).

The semi-pure methylsulfomycin 1, thus obtained, was further purified by two successive preparative HPLCs on a [16 mm x (30 + 120) mm] ODS-Hypersil^{®} (5µ) column. The eluant was methanol - water (68 : 32) at a flow rate of 9 ml/min and detection at 210 nm. Thus, purification of 1.1 g of semi-pure material yielded 110 mg of pure methylsulfomycin I.

The physico-chemical and spectral properties of methylsulfomycin I are summarized in Table 1. The minimum inhibitory concentration (MIC) against different test strains, determined by agar dilution method in the known manner, are given in Table 2.

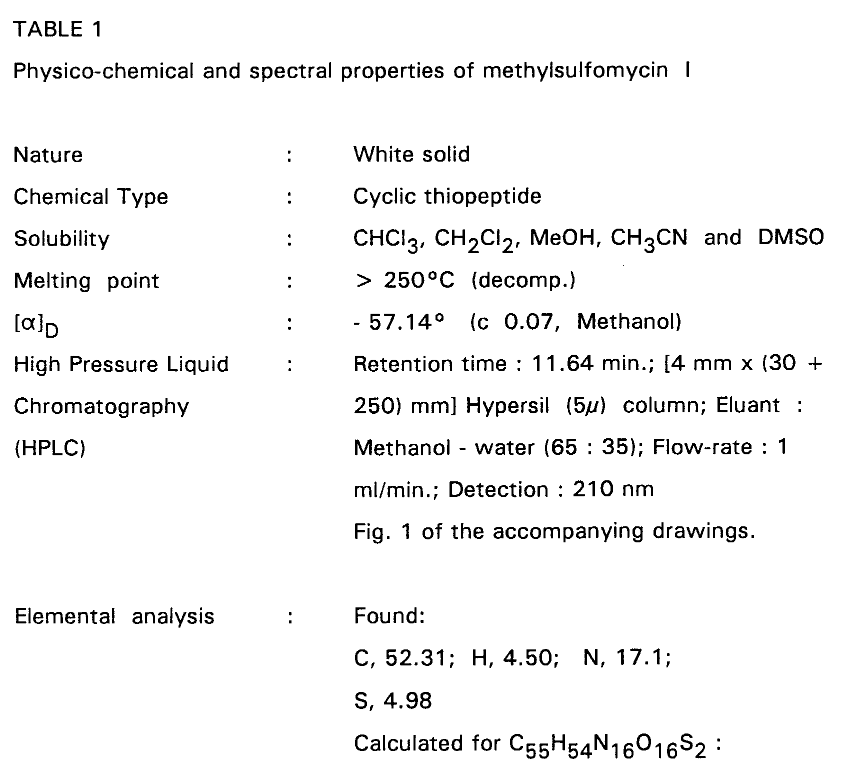

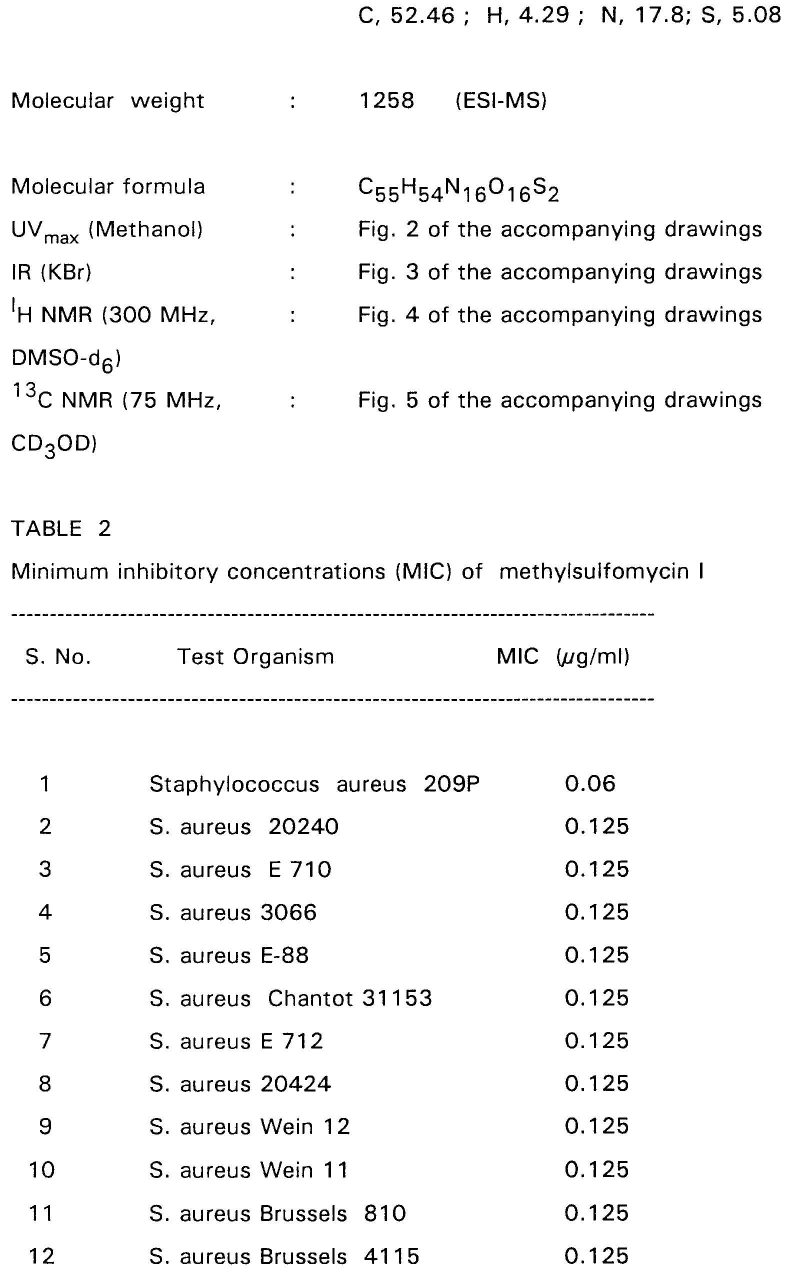

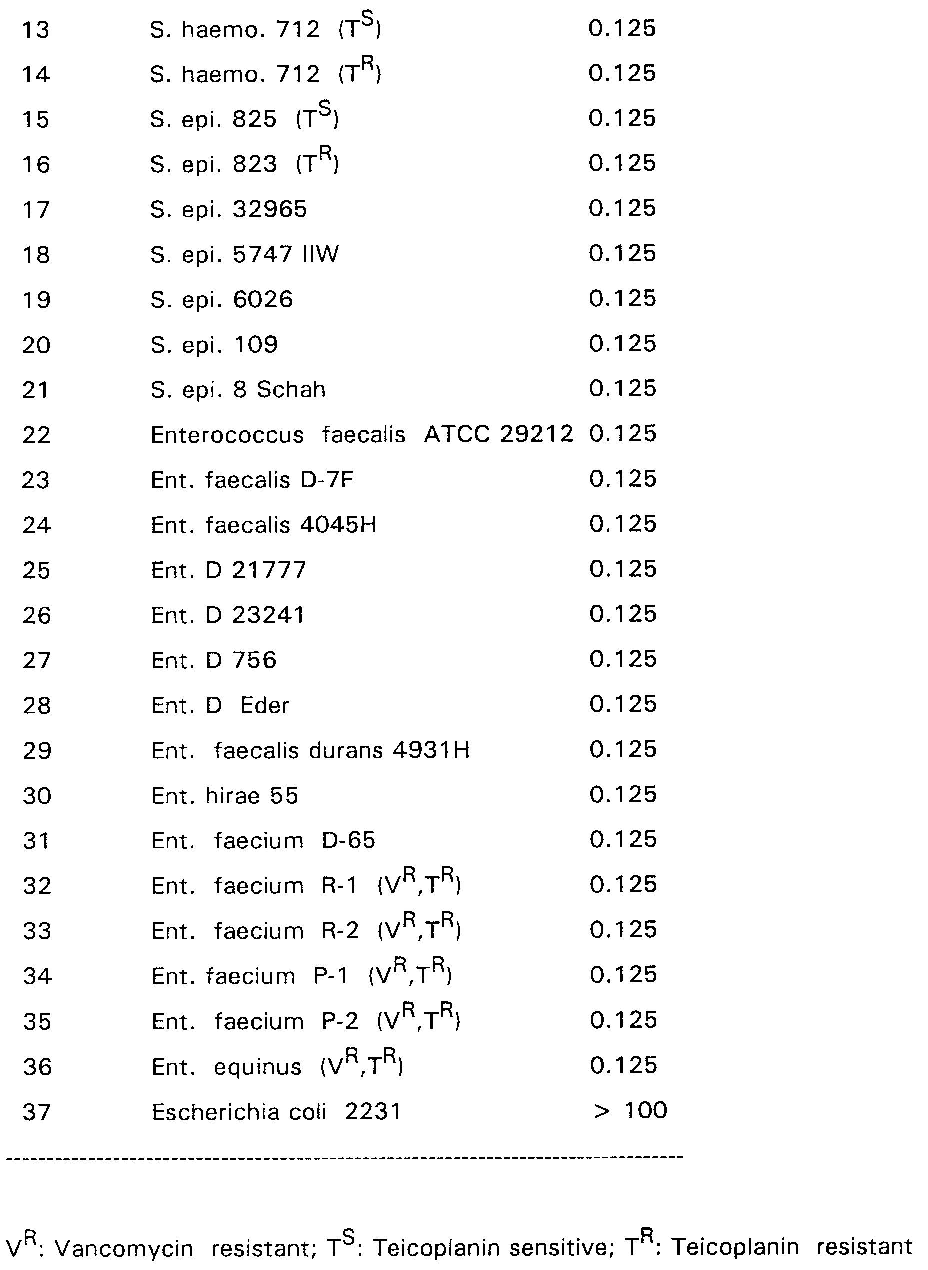

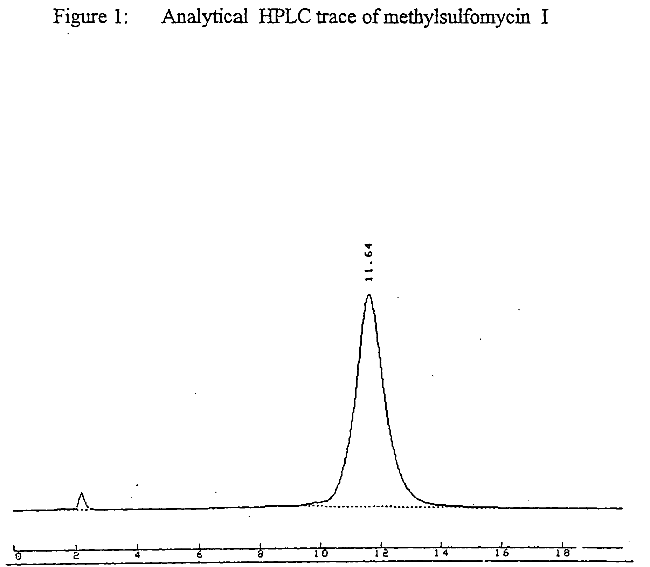

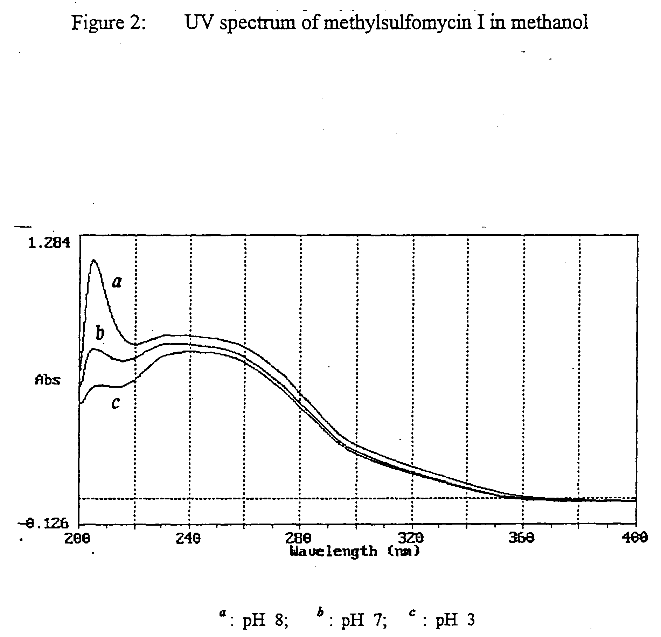

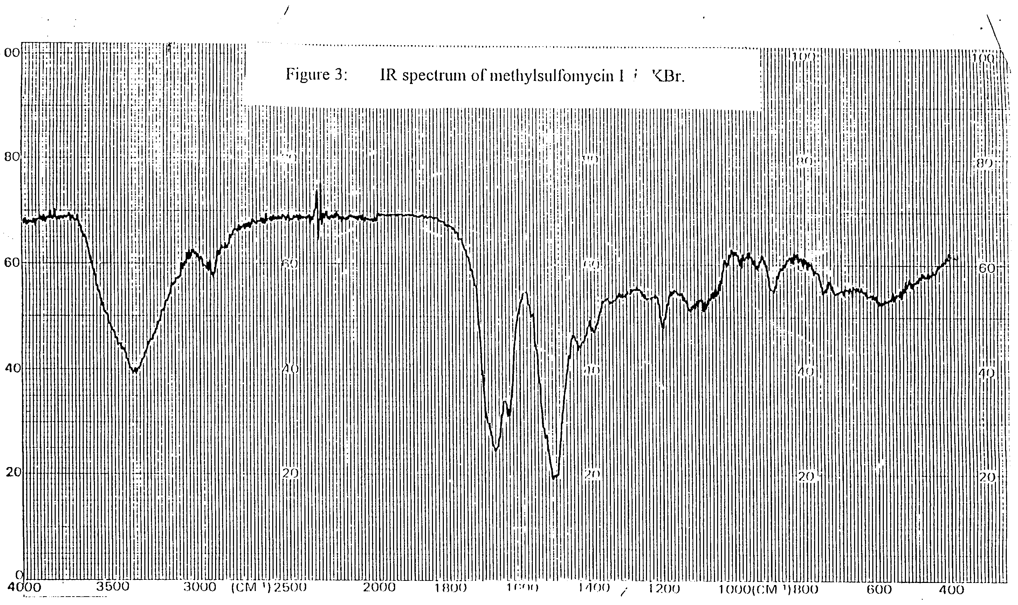

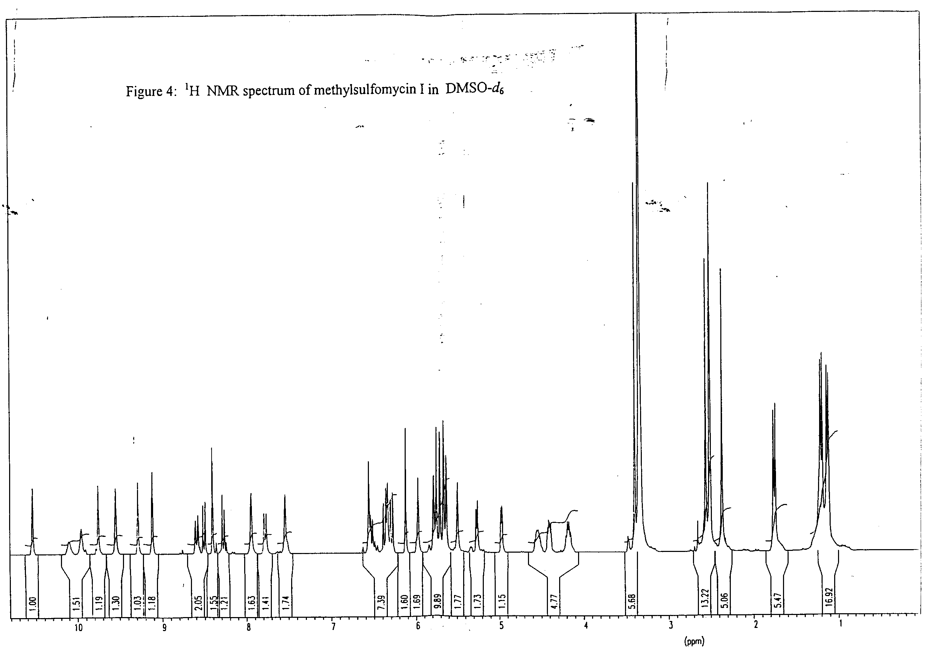

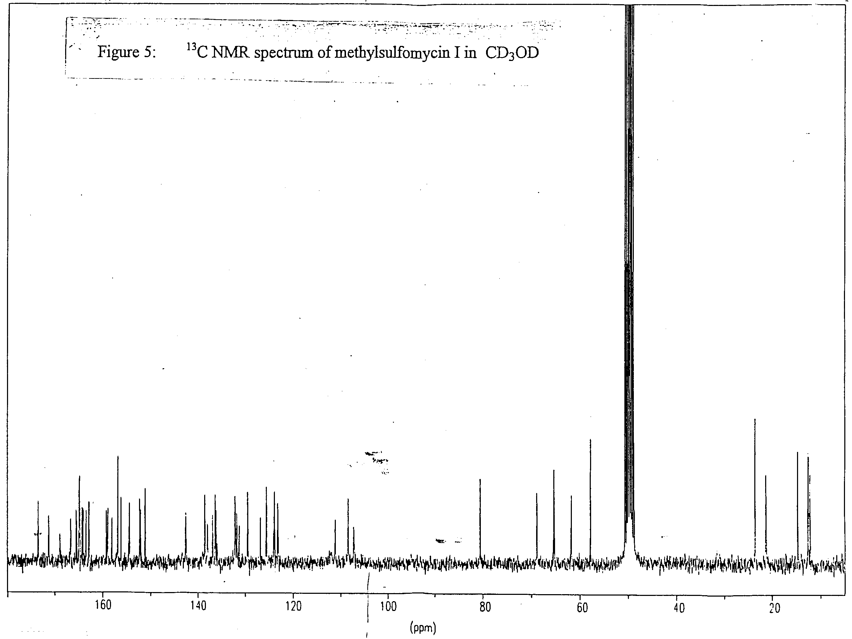

## Claims

1. Methylsulfomycin I, a compound of the molecular formula C₅₅H₅₄N₁₆O₁₆S₂ as well as the pharmaceutically acceptable salts thereof.

2. Methylsulfomycin I, a compound of the molecular formula C₅₅H₅₄N₁₆O₁₆S₂ obtainable by actinomycete species HIL 006044 (DSM 11008) producing microorganism and the subsequent isolation and purification from the culture broth, as well as the pharmaceutically acceptable salts thereof.

3. A process for the preparation of methylsulfomycin I as claimed in claims 1 or 2, which comprises actinomycete species HIL 006044 (DSM 11008), the mutants and/or variants thereof, being cultivated under aerobic conditions in a nutrient medium containing sources of carbon and of nitrogen, inorganic nutrient salts and trace elements, and the compound being isolated and purified from the culture broth in a customary manner.

4. The process as claimed in claim 3, wherein the cultivation is carried out at temperatures between about 28 and 32°C and at a pH between about 6 and 8.

5. The process as claimed in claims 3 or 4, wherein the cultivation is carried out at 29°C ( 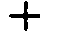 1°C) and a pH about 7.0.

6. The process as claimed in one or more of claims 3 to 5, wherein the fermentation is carried out as submerged fermentation.

7. A pharmaceutical which contains a compound as claimed in claims 1 or 2, where appropriate, in addition to auxiliaries and/or excipients customary for the preparation of pharmaceuticals.

8. The use of the compound as claimed in claims 1 or 2 as a pharmaceutical.

9. The use of a compound as claimed in claims 1 or 2 for the production of a pharmaceutical having an antibiotic action.

10. Actinomycete species HIL 006044 (DSM 11008).
